# EUROPEAN PATENT SPECIFICATION

(11) **EP 2 902 031 B1**
(45) Date of publication and mention of the grant of the patent: **24.10.2018**
(21) Application number: 15158919.9
(22) Date of filing: 08.12.2009
(51) Int. Cl.: A61K 31/573, A61K 33/00, A61K 38/17, A61K 38/18, A61K 45/06, A61P 1/00, A61P 29/00

(54) **TOPICALLY ACTIVE STEROID FOR USE IN RADIATION INJURY**
TOPISCH AKTIVES STEROID ZUR VERWENDUNG BEI STRAHLENSCHÄDEN
STÉROÏDE ACTIF PAR VOIE TOPIQUE POUR UNE UTILISATION DANS DES RADIOLÉSIONS

(30) Priority: 08.12.2008 US 120785 P
(43) Date of publication of application: 05.08.2015
(62) Divisional of application: 09836727.9
(73) Proprietor: Soligenix, Inc., Princeton, NJ 08540 (US)
(72) Inventor: Brey, Robert N., Princeton, NJ New Jersey 08540 (US); McDonald, George B., Bellevue, WA Washington 98004 (US); Schaber, Christopher, Princeton, NJ New Jersey 08540 (US)
(74) Representative: Winter, Brandl, Fürniss, Hübner, Röss, Kaiser, Polte - Partnerschaft mbB

(56) References cited:
- WO-A1-2008/101215
- US-A- 4 661 509
- US-A1- 2002 086 857
- US-A1- 2003 060 486
- US-A1- 2003 129 687
- ABDELAAL A S ET AL: "Treatment for irradiation-induced mucositis.", LANCET 14 JAN 1989 LNKD- PUBMED:2562899, vol. 1, no. 8629, 14 January 1989 (1989-01-14), page 97, XP002674726, ISSN: 0140-6736
- KORKUT CAGATAY ET AL: "Histopathological comparison of topical therapy modalities for acute radiation proctitis in an experimental rat model.", WORLD JOURNAL OF GASTROENTEROLOGY : WJG 14 AUG 2006 LNKD- PUBMED:16937473, vol. 12, no. 30, 14 August 2006 (2006-08-14), pages 4879-4883, XP002674727, ISSN: 1007-9327
- CAVCIC JOSIP ET AL: "Metronidazole in the treatment of chronic radiation proctitis: Clinical trial", CROATIAN MEDICAL JOURNAL, vol. 41, no. 3, September 2000 (2000-09), pages 314-318, XP002674728, ISSN: 0353-9504

## Description

### TECHNICAL FIELD OF THE INVENTION

Treatment and prevention of tissue damage resulting from acute radiation injury in the gastrointestinal tract with locally effective therapeutic agents.

### BACKGROUND OF THE INVENTION

The combination of radiotherapy and chemotherapy is often used for the eradication of malignant cancer cells. Exposure to radiation or chemotherapeutic agents often results in the destruction of normal tissue, especially hematopoietic cells and epithelial cells in the gastrointestinal tract. Further, damage to normal tissues can also result from environmental sources of radiation that include accidental exposure to radiation or contact with radionuclides in, for example, the release of radioactive material following a nuclear accident or the purposeful use of "dirty" bombs during a terrorist attack.

Although radiation by itself or in combination with chemotherapy is effective in targeting the malignant cells, or treating other diseases, such use is typically associated with damage to normal cells and tissue. This damage can include fibrosis, vascular damage, aberrant angiogenesis, pneuminitis, atherogenesis, osteonecrosis, immunosuppression and functional impairment of the gastrointestinal tract, lungs, kidneys, and other organs. Epithelial cells in the upper and low gastrointestinal tract are especially vulnerable to damage by radiation and chemotherapeutics agents used in cancer, and by analogy to radiation in the form of environmental exposure to radionuclides or high dose radiation from accidental or purposeful exposure. Damage to the epithelial tissue, directly and indirectly can lead to gastrointestinal symptoms and chronic conditions including mucositis, enteritis, and proctitis.

The epithelial tissue of the mouth and esophagus are particularly sensitive to chemotherapy and radiation. For example, after radiation treatment for head and neck cancers, oral ulcerations characteristic of mucositis are a major clinical problem causing considerable pain, increased susceptibility to infection and inability to eat. Treatment of abdominal or pelvic cancers with radiation causes radiation enteritis, damage to the intestinal lining occurring typically in the small bowel, and less frequently in the large bowel. In the case of radiation therapy of cancer localized in the pelvis or abdomen, such as ovarian cancer, radiation enteritis is one of the most difficult to treat complications of abdominal and pelvic radiation. It is thought, for example, radiation treatment or exposure causes depletion of epithelial cells with each successive dose of pelvic radiation and/or chemotherapy which results in the clinically observed acute gastrointestinal symptoms. The incidence of radiation enteritis is increasing because of the current trend of combined high dose chemotherapy and high dose radiation. By an equivalent process, localized radiation treatment of prostate cancer results in lower bowel proctitis. An almost identical process of intestinal and epithelial injury can occur with use of certain chemotherapeutic agents, such as 5-fluorouracil, cisplatin, methotrexate, doxorubicin, hydroxyurea, cytosine arabinoside, and irinotecan. Injury to the gastrointestinal tract following radiation therapy, chemotherapy or accidental exposure to ionizing radiation, resulting in enteritis, mucositis, or proctitis, has a significant role in patient survival or mortality as well as quality of life.

Mucositis, proctitis or enteritis occurs by several mechanisms and point to a multifactorial cause for the resulting ulcerations and symptoms, including direct damage to the epithelium by anticancer drugs or radiation. Indirectly, increased epithelial damage during prolonged radiation exposure or exposure to chemotherapeutic agents is a result of inflammatory responses caused by infiltration lymphocytes, neutrophils and macrophages and concomitant secretion of pro-inflammatory cytokines and other cellular effector molecules. Immediate post radiation exposure is accompanied by persistently elevated levels of pro-inflammatory cytokines such as TGF-β and chemokines, such as RANTES, MCP-1, and IL-10. Chemokines are chemotactic peptides that specifically recruit inflammatory cells after cellular injury. The chronic elevation of the specific chemokines may explain the persistence of mononuclear cells in the fibrotic phase of injury. In the long term, the damage can result in fibrosis and multi organ failure. In short term following radiation or chemotherapy exposure, cellular damage is evident. In the gastrointestinal tract, cellular changes are typified by a process of programmed cell death (apoptosis). Such cellular changes as a result of radiation-and/or chemotherapy-induced apoptosis include shortening of villus tips; reduction in total epithelial surface area; reduction or disappearance of the glycocalyx and loss of pluripotent cells. Apoptosis of intestinal villi and reduction of their replenishment from the stem cell compartment leads to breach of the intestinal epithelium.

Because hematopoietic and epithelial cells are highly sensitive to radiation damage, their loss after radiation exposure or chemotherapeutics uses often results in lethal infections on several accounts. Direct damage to the gut causes the breach of epithelial barriers to allow pathogens to enter. The acute effects of ionizing radiation on the intestinal mucosa are generally ascribed to inhibition of epithelial mitosis in the crypts. Renewal of the intestinal epithelial barrier depends upon an active stem cell compartment similar to the hematopoietic system, and the intestinal stem cells are particularly sensitive to ionizing radiation exposure. With increasing radiation dose, the intestinal stem cells cannot produce enough cells to repopulate the villi, which results in blunting and diminution in villus height and eventual functional incapacity. This leads to decreased nutrient absorption and barrier function and bacterial translocation through the intestinal barrier.

Direct damage to epithelial cells also results in hypoperfusion of the intestine, that is, loss of fluid and electrolytes. Persistent gut hypoperfusion is an important inciting event in the development of the systemic inflammatory response syndrome and multi-organ failure (MOF). Increased intestinal vascular permeability together with capillary leakage has been observed in the early period after irradiation, accompanied by several post-irradiation alterations including moderate to marked dilatation, shortening and tortuosity of small arterial vessels, reduction in numbers and/or lengths of vessels and later occurring hemorrhagic patterns. The endothelial layer is also affected. Endothelial apoptosis also plays a key role in the loss of the intestinal mucosa and survival.

Radiation also affects circulating cells in the immune system. The destruction of cells in the blood involved in the innate and adaptive immune response results in neutropenia, a state of compromised immune system in such patients. As a result, any drug or treatment that causes significant neutropenia can precipitate enteritis or mucositis.

Symptoms of both acute radiation and/or chemotherapy-induced enteritis damage include bowel injuries that result in fistulas, strictures, ulceration, perforations, and chronic malabsorption. Symptoms of nausea, vomiting and anorexia may also be experienced which results in a general nutritional wasting leading to severe diarrhea and pain. The symptoms are potentially life threatening complications and have an impact on patient quality of life. Cachexia and death may ensue.

Functional changes in the small bowel, induced by pelvic irradiation and/or chemotherapy include malabsorption of fat, carbohydrates, protein, and bile salts, and clinically presents as diarrhea. The segment of the small intestine most affected by pelvic radiation is the ileum, and this is due to its pelvic position which puts it directly into the field of the radiation beam. Radiation enteritis is characterized by diffuse collagen deposition and progressive, occlusive vasculitis. The vasculitis and fibrosis progress over time, resulting in narrowing of the intestinal lumen with dilation of the bowel proximal to the stricture. On a chronic basis, radiation exposure leads to tissue fibrosis, depending on the location of the radiation injury. The fibrotic process is progressive, resulting in worsening of clinical symptoms. The affected segments of intestine and serosa become thickened. In the case of damage to the intestine, the affected area is often surgically resected. Even so, the fibrotic process may continue over time despite surgery to remove the affected region. Ulceration, necrosis, and occasional perforation of the intestinal wall may occur. The sigmoid colon is also affected by pelvic radiation treatment due to its pelvic position. The onset of chronic pelvic radiation injury may be delayed for months to years. Medical management of late bowel damage is difficult and these late complications of radiation therapy can have an adverse effect on the host's nutrition status. The same scenario holds true for chronic chemotherapy-induced injury to the gastrointestinal tract.

Rapidly proliferating tissues, such as small intestine crypt cells, are particularly sensitive to radiation and concurrent administration of chemotherapy. Radiation does not inhibit the migration of epithelial cells out of the crypts and up the villi, the process of regeneration per se. Rather, they undergo apoptosis and are shed from the intestine villus. After intestinal epithelial crypt cells are exposed to irradiation, mitotic arrest results in an increase in the ratio of immature secretory crypt cells to mature villous enterocytes. A shift in balance between the number of absorptive and secretory cells and destruction of the brush-border enzymes involved in the terminal digestion of carbohydrates, fats, proteins, and bile salts leads to abnormal absorption and secretion of fluids and electrolytes. Under normal physiologic conditions, both small intestine and colonic epithelium undergo a low rate of spontaneous apoptosis. Rapid increase in the rate of apoptosis of the intestinal crypts occurs when the animals are exposed to low-dose radiation (1-5 centigrays [cGy]). Apoptosis is usually observed mainly in the stem cells of the crypts. The rate of apoptosis was dose dependent and reached a plateau at 1 Gray (Gy). Parallel to the increased rate of apoptosis is an increased expression of the tumor suppressor gene *p53* in the stem cell region. Apoptosis induced by radiation is dependent on the presence of *p53.* Small intestinal stem cells are more sensitive to radiation compared with stem cells in the colon and rectum because of the presence of *bcl-2* in the latter.

Treatment of epithelial damage and associated mucositis, enteritis, and proctitis has been attempted with a variety of therapeutic molecules, though none has been shown to be completely effective alone or with any other compound to treat symptoms due to radiation injury. In the case of radiation or chemotherapy-induced enteritis, the most common treatment approach has been the use of nonspecific agents. Several agents are currently used in common practice, including paregoric, the diphenoxylate and atropine, and loperamide. The purpose of use of those drugs to mitigate the symptoms of diarrhea and not the underlying causes of epithelial damage and inflammation. Although partially effective in ameliorating the symptoms, the utility of these drugs is not thought to be useful in the control of more serious symptoms, and are merely palliative. Other agents that have been used in clinical studies include as nonsteroidal anti-inflammatory agents, bismuth subsalicylate, cholestyramine. Other treatment and prevention strategies have also been employed in animal studies and in the clinic. For example, the somatostatin analogue octreotide has been shown to have some benefit in ameliorating the diarrheal symptoms of radiation enteritis because of direct effects on chloride secretion, but likely has little role in preventing epithelial damage due directly to irradiation and indirectly due to inflammation. For example, it is thought that octreotide reduces acute mucosal changes after irradiation of small bowel because of inhibition gastrointestinal hormones which in turn affects motility, blood flow, and epithelial cell proliferation. The upregulation of the gastric hormone gastrin is associated with a higher degree of radioprotection. Other examples of therapeutic strategies employed to treat the effects of radiation injury include antioxidants, such as vitamin E, either alone or in combination with pentoxyfylline, treatment with hyerbaric oxygen, modification of diets, including the use of probiotics to compete with intestinal pathogens, inhibitors of cycooxygenase-2 (Cox-2) such as Rofecoxib (Celebrex®), inhibitors of the LPA2 receptors, Amifostine (Ethylol), and formulations of drugs such as micronized sucralfate.

The current conventions and method that are becoming common to treat radiation damage in particular incorporates the concept of supportive care to counteract the effects of neutropenia by treating patients with GMCSF to bolster the replenishment of neutrophils, or to counteract the effects of bacterial pathogenesis with antibiotics. In addition, supportive care is the use of methods to reconstitute the blood compartment with autologous or heterologous bone marrow. Supportive care such as described does not treat inflammation directly and does not intend to treat the epithelial damage that underlies the primary lesions.

It has become increasingly evident that specific therapeutic interventions are needed to address the multifactorial nature of the damage cause; this includes strategies to mitigate the damage to the epithelium itself by the use regulatory molecules that are intended to propagate the process of epithelial cell regeneration, anti-inflammatory agents that are aimed to reduce the infiltration of monocyte, neutrophils, and the resultant secretion of proinflammatory cytokines and chemokines, or to mitigate the proinflammatory effects of microbial breach of epithelial barriers. There is still a need for improved therapy to treat the consequences of radiation exposure either in the form of controlled measured radiation therapy or radiation, perhaps high dose exposure due to accidental exposure to radiation sources or exposure as consequence of a dirty bomb or nuclear accident.

Other therapeutic interventions can be considered to treat or prevent epithelial damage from excess radiation or chemotherapy. For example, keratinocyte growth factor (KGF) has been shown to be a cytoprotective agent against chemoradiotherapeutic toxicities. KGF binds to a splice variant of fibroblast growth factor receptor 2 (FGF-2) known as the KGF receptor (KGFR). Epithelial tissues are the only tissue type known to express KGFR. When KGF binds to KGFR in the oral mucosa it acts through a tyrosine kinase-mediated pathway to enhance cell proliferation. In murine models, KGF can also ameliorate GVHD and idiopathic pneumonitis after allogeneic bone marrow transplantation (BMT). KGF reduced immune mediated injury of damaged epithelial tissue. KGF has proliferative effects on epithelial cells, and protects epithelial cells from injury induced by chemotherapy, radiation and oxidative stress. KGF protects thymic epithelial cells from radiation. BMT recipient mice were given KGF or placebo prior to TBI and allogeneic BMT. KGF pretreatment increased the capacity of the thymus to generate donor-derived thymocytes, increased the number of naive T cells in the peripheral blood and improved the immune response to neoantigen. KGF treatment increased production of intrathymic IL-7. KGF prevented thymic injury and prolonged immune deficiency in BMT recipients. KGF also augmented immune reconstitution in rhesus macaques after autologous HCT.

A clinical trial of KGF for the prevention of oral mucositis after high-dose radiation has shown that patients who received rhKGF 60 µg/kg/day for 3 days prior to a 12 Gy fractionated TBI conditioning regimen and 3 days after infusion of autologous peripheral blood stem cells (PBSC) experienced a significant decrease in the severity of mucositis and duration of mucositis compared to placebo, 3.4 days versus 10.4 days, respectively, p<0.001. The incidence of febrile neutropenia was 26% for KGF versus 46% for placebo, and the mean days of febrile neutropenia was 2.6 days for KGF versus 4.6 days for placebo. KGF is currently used for patients across the U.S. to prevent chemoradiation induced mucositis. KGF is safe, without significant risk to healthy volunteers, and has predictable pharmacokinetics. However, the effectiveness of KGF in radiation induced enteritis or injury had not been demonstrated.

Lithium, a simple cation has been used for the treatment of bipolar disorder, but is also known to have effects on morphogenesis in early development. For example, lithium potently and specifically inhibits glucagon synthetase kinase 3-beta (GSK-3β) activity), which activates intestinal stem cell proliferation via the *wnt* /β catenin signaling pathway, playing a critical role in intestinal stem cell proliferation and development. By inhibiting GSK-3β, lithium permits upregulation of Wnt/β-catenin, which stimulates intestinal crypt stem cell proliferation. Lithium has been administered to grey collie dogs for the successful treatment of cyclical neutropenia. Lithium also enhanced increased granulocyte recovery and decreased cyclophosphamide-induced gut injury in the dog. Treatment of mice with lithium results in enlarged crypts, however the effects of lithium on radiation-induced epithelial injury were not known.

With regard to the Wnt pathway, other regulatory peptides and hormones may be useful for the treatment of radiation induced injury. For example, R-spondin1 mediates restoration damaged intestinal epithelium, most likely through the Wnt pathway. R-Spondin (RSpo) family of secreted ligands that, similar to canonical Wnt family members, activate β-catenin signaling. The R-spondin protein family includes four human paralogs (R-spondin1-4), each of which contains a leading signal peptide, two cysteine-rich, furin-like domains, and one thrombospondin type 1 domain. The Wnt family of secreted proteins plays a critical role including development, differentiation, and proliferation of intestinal epithelial cells. Wnt signaling induces downstream cellular responses by regulating cytosolic levels of β-catenin. In the absence of Wnt, cytosolic β-catenin is phosphorylated and targeted for rapid degradation by the proteasome. Wnt induces sequential phosphorylation of PPPSP motifs in the LRP6 cytosplasmic tail by GSK3 and casein kinase I and subsequent recruitment of the scaffold protein Axin.

Locally acting anti-inflammatory drugs, for which the main pharmacological actions take place locally in the epithelial tissue of the alimentary canal can act to reduce the local effects of immune activation and inflammation. This has been demonstrated in the case of Crohn's disease with budesonide (Entocort®), being partially effective in inducing Crohn's remission, and for intestinal graft versus host disease for beclomethasone dipropionate (BDP). Along with budesonide and similar topically acting corticosteroid drugs (TACs), BDP is a potent, locally-acting corticosteroid. For example, the anti-inflammatory and immunosuppressive effects of BDP and its metabolite, 17-beclomethasone mono-propionate (17-BMP), are considered to be its primary pharmacologically active metabolites. The anti-inflammatory activity of BDP results from both its genomic effects (repression of transcription factors leading to reduced synthesis of pro-inflammatory cytokines, inhibition of expression of adhesion molecules, and apoptosis of T cells), as well as potent non-genomic effects (immune suppression, apoptosis of T cells), and intracellular global regulatory molecules such as NFK-β.

The major degradation pathway of BDP in human plasma has been proposed to be from BDP to 17-BMP and 21-BMP (with interconversion between these 2 metabolites), and then to beclomethasone (BOH). The BDP metabolite 17-BMP is ∼25 times more potent than BDP itself and has glucocorticoid receptor binding affinity 13 times that of dexamethasone. BDP and BOH bind to the glucocorticoid receptor with approximately half and three-fourths, respectively, of the binding affinity of dexamethasone, and 21-BMP has no apparent affinity for the receptor. In addition to having greater binding affinity to glucocorticoid receptors, 17-BMP has higher apparent variability than the other metabolites. Unchanged BDP was not detected in the plasma of rats following oral administration of ³H-BDP. The primary metabolite (17-BMP) was found in the plasma of the rats, suggesting rapid transformation of parent to metabolite in gut, plasma, or both. Intravenous administration of ³H -BDP resulted in the brief appearance of unchanged BDP with a half-life of 3-4 minutes, and the immediate appearance of high concentrations of 17-BMP suggesting rapid transformation of the parent to the metabolite within plasma. Hydrolysis of BDP to 17-BMP is also likely to occur rapidly in the intestines, with further hydrolysis from 17-BMP to BOH at a very slow rate. The half-lives of BDP and 17-BMP in simulated intestinal fluid were 2.1 minutes and 12 hours, respectively. Hydrolysis of BDP to 17-BMP may occur in both intestinal fluid or in mucosal epithelial cells. The combination of avid glucocorticoid receptor binding affinity, high tissue concentration, prolonged residence time in the GI mucosa, and enterohepatic circulation of 17-BMP suggest that oral BDP provides for significant topical activity in the mucosa of the GI tract. In addition, a relatively low absolute oral bioavailability of 17-BMP after DBP administration, estimated at 21-41%, with high clearance, limits systemic exposure. Topical BDP has been used to decrease skin desquamation following breast irradiation and inhaled BDP has been used to decrease pneumonitis after thoracic irradiation. BDP enemas have been successfully used for treatment of distal ulcerative colitis. Other potent corticosteroids applied as topical agents have been successful for treatment of acute radiation dermatitis. However, it was not known that BDP or other topical steroids or anti-inflammatory drugs applied locally to epithelial tissue in the alimentary canal would treat or prevent inflammatory damage epithelial due to radiation damage either alone or together with drugs and molecules that would act other points in the multifactorial process.

While significant advances have been made with regard to the treatment of supportive care during radiation injury, there is still a need in the art for improved methods for treating radiation injury by controlling the multifactorial causes of the underlying tissue damage. It is the surprising discovery of this invention that the topically active corticosteroid drug BDP acts alone to reduce the inflammatory cascade and the symptoms of high dose radiation injury and can be used to treat and prevent radiation enteritis, mucositis, and radiation injury to epithelial tissue upon topical application. Further, the surprising unanticipated discovery of this invention is that BDP acts synergistically with growth factors and cytokines to further reduce the duration and intensity of symptoms arising form radiation induced tissue damage in gastrointestinal epithelial tissue and can protect against the development of ulcerations and tissue damage if given prior to exposure. It is additionally the surprising discovery of this invention that BDP and lithium act additively or synergistically to reduce the severity, onset, and duration of symptoms.
US 2003/0129687 A1 relates to the therapeutic use of Fibroblast Growth Factor 12 to promote or accelerate wound healing such as oral mucositis.

By this present invention, total body irradiation (TBI) injury can be treated or prevented with the topically active corticosteroid BDP in the gut lumen, either as a monotherapy or synergistically with growth factors, such as KGF or R-spondin-1, in addition to supportive care that is intended to replenish neutrophils (e.g. GMCSF) and prevent bacteremia (antibiotics). Topical BDP can reduce the inflammatory cytokine storm induced by the radiation damaged GI tract. Gut injury from irradiation is associated with epithelial cell damage and hypoperfusion of the intestine. This, in turn, stimulates a systemic inflammatory response. BDP and its metabolite 17-BMP within the gut mucosa can inhibit the cellular and innate immune mechanisms that exaggerate mucosal damage.

### SUMMARY OF THE INVENTION

The present invention provides a novel pharmaceutical composition for use in preventing, ameliorating and/or treating damage to the lining of the alimentary tract of a patient by administering an effective amount of a topically active corticosteroid, wherein the damage is a result of the patient's exposure to total body irradiation, the topical active corticosteroid is beclomethasone dipropionate, the effective amount of beclomethasone dipropionate is 4 mg/day, and the administration of beclomethasone dipropionate is carried out from 2 hours after the total body irradiation.

The present invention is based on the discovery also that such BDP in orally administered formulations acts synergistically or additively with growth factors and other therapeutic molecules that function in repairing epithelial damage and mitigate the symptoms of radiation-induced damage in the gastrointestinal tract or oral cavity. The compounds and drugs include keratinocyte growth factor (KGF), R-spondin-1 and its analogues and homologues, hormones such as somatostatin, octreotide, gastrin, Ghrelin, inhibitors of cyclo-oxgenase -2, antioxidants, vitamin E, sucralfate, analogues of lysophosphatidic acid and agonists or antagonists of LPA-2 receptor, amifsotine and other radioprotectant or radio-mitigating drugs.

An effective dose of the selected topically active corticosteroid drug (i.e. BDP) can be introduced into the oral cavity or to the gastrointestinal tract as an oral agent and that only a fraction of the dose is absorbed into the systemic circulation and the fraction that is absorbed provides for limited systemic exposure and is rapidly eliminated. The potent anti-inflammatory activity of the corticosteroid drug is concentrated specifically in the affected areas of the oral cavity or the gastrointestinal tract. Side effects usually attributed to systemic steroids are therefore minimized, while controlling the tissue inflammation and ulceration. Venous blood draining from the small and large bowel eventually passes into the liver through the portal vein. So long as the dose of the selected steroid drug used is not excessive, the liver appears to be capable of inactivating the drug it receives, before significant amounts build up in the systemic circulation.

Thus according to the present invention, there is provided an oral composition for administration as topical treatment for a patient that is subjected to TBI induced injury appearing in the gastrointestinal tract or the oral cavity. In a preferred embodiment of this invention, the TAC is in an oral formulation that releases the drug locally into the upper gastrointestinal tract, particularly the small bowel.

### DETAILED DESCRIPTION OF THE INVENTION

### Definitions:

By "an effective amount" is meant a quantity of topically active corticosteroid which will upon single or multiple dose administration to the patient be effective in the prophylaxis, amelioration and/or treatment of damage to the lining of the alimentary tract resulting from radiation.

By "preventing, ameliorating and/or treating" is meant a reduction or elimination of subsequent damage compared with the damage which would have occurred if the corticosteroid were not administered; and in the case where the corticosteroid is administered after the damage has occurred, a reduction or elimination of such damage.

By "damage" is meant any alteration in normal structure or function. Such damage includes mucosal inflammation-mucositis and enteritis and also a partial loss of mucosal crypt area and/or mucosal villus length, or an increase in bacterial translocation across the alimentary tract.

The term "alimentary tract" as used herein refers to the digestive passage in any animal from mouth to anus and includes mouth, esophagus, stomach and intestines (including small and large bowel). In a preferred aspect, the present invention is particularly applicable to the small bowel.

By "lining" is meant any biological material which covers a surface or lines a cavity or the like and which performs protective, screening and/or other functions. The lining of the alimentary tract includes the oral, esophageal and gastrointestinal epithelia.

By "topically active" or "locally active", is meant the compound has its principal pharmacological action through tissue near the site where the drug is present. In the case of TAC, active in the gastrointestinal epithelium, TAC drugs are absorbed in the intestinal epithelial tissue and have their primary action on epithelial cells, whereas the TAC has limited systemic exposure either by limited absorption, first pass metabolism by the liver and/or gut, enterohepatic recirculation, protein blinding, or rapid elimination, and any combination thereof.

By "systemic circulation" it is meant that portion of the circulation which is distal to the site of steroid drug metabolism, in which a steady-state level of the drug in the circulation has been achieved.

By a "pharmaceutically acceptable carrier or excipient" is meant a carrier or excipient which is compatible with the other ingredients of the composition and not injurious to the patient.

The term "effective amount" means that amount of a drug or pharmaceutical agent that will elicit the biological or medical response of a tissue, system, animal or human that is being sought by a researcher or clinician.

The term "therapeutically effective amount" means any amount which, as compared to a corresponding subject who has not received such amount, results in improved treatment, healing, prevention, or amelioration of a disease or disorder, or a decrease in the rate of advancement of a disease or disorder, and also includes amounts effective to enhance normal physiological function.

By "supportive care" is meant therapy to counteract the effects of neutropenia/ by treating patients with GMCSF to bolster the replenishment of neutrophils, or to counteract the effects of bacterial pathogenesis with antibiotics. In addition, supportive care is the use of methods to reconstitute the blood compartment with autologous or heterologous bone marrow.

Although it is preferred to treat patients with the TAC drug in the practice of the present invention via oral administration for treating/preventing/reducing the severity of enteritis incident to TBI injury, and via mouthwash formulation or lozenge for preventing/reducing the severity of mucositis incident to radiation, the topically active corticosteroids in the practice of the invention can be otherwise administered in buccal and sublingual dosage forms as tablets, capsules (each including timed release and sustained release formulations), pills, powders, granules, elixirs, tinctures, suspensions, syrups and emulsions.

Likewise, the TAC drug BDP may also be administered in nasal, ophthalmic, otic, rectal, intravenous (both bolus and infusion), intraperitoneal, intra-articular, subcutaneous or intramuscular inhalation or insufflation form, all using forms well known to those of ordinary skill in the pharmaceutical arts.

The dosage regimen utilizing the compound of the present invention is selected in accordance with a variety of factors including type, species, age, weight, sex and medical condition of the patient; the severity of the condition to be treated; the route of administration; the renal and hepatic function of the patient; and the particular compound or salt thereof employed. An ordinarily skilled physician can readily determine and prescribe the effective amount of the drug required to combat the enteritis and/or mucositis condition.

In the case of combination therapy, the TAC is preferably administered in an oral dosage form in as described, and the co-drug is administered either orally in direct combination or concomitantly with the oral TAC as an intravenous preparation. In one preferred embodiment the combination therapy consists of BDP in an oral dosage form with an intravenous effective dosage amount of R-spondin-1. The preferred dose of R-spondin-1 will range between 5 to about 1000 micrograms/kg of body weight, and in particular about 10 to about 100 micrograms of body weight.

In one other preferred embodiment of the combination, the combination therapy consists of BDP in an oral dosage form with an intravenous dosage amount of KGF. The preferred dose of KGF will range between 0.1 microgram/kg body weight to about 1 mg; in a more preferred embodiment of the invention the dosage range of KGF is between 1 and about 60 micrograms/kg body weight.

The dosage to be administered is based on the usual conditions such as the physical condition of the patient, age, body weight, past medical history, route of administrations, severity of the conditions and similar considerations. In some cases, a relatively lower dose is sufficient and, in some cases, a relatively higher dose or increased number of doses may be necessary. Oral administration may be once or more than once per day depending on the course of radiation. Advantageously, the compound of the present invention may be administered in a single daily dose, or the total daily dosage may be administered in divided doses of two, three or four times daily. The compound for use according to the present invention can be prepared in a range of concentrations for topical use of about 0.1 to about 5 mg/ml of suitable solvent.

For post exposure radiation treatment, administrations are as needed. Furthermore, the compound of the present invention can be administered in intranasal form via topical use of suitable intranasal vehicles, or via transdermal routes, using, those forms of transdermal skin patches well known to those of ordinary skill in that art.

In the present invention, BDP is typically administered in admixture with suitable pharmaceutical diluents, excipients or carriers (collectively referred to herein as "carrier" materials) suitably selected with respect to the intended form of administration, that is, oral tablets, capsules, elixirs, syrups and the like, and consistent with conventional pharmaceutical practices.

For instance, for oral administration in the form of a tablet or capsule, the active drug component can be combined with an oral, non-toxic pharmaceutically acceptable inert carrier such as ethanol, glycerol, water and the like. Powders are prepared by committing the compound to a suitable fine size and mixing with a similarly comminuted pharmaceutical carrier such as an edible carbohydrate, as, for example, starch or mannitol. Flavoring, preservative, dispersing and coloring agent can also be present.

Capsules are made by preparing a powder mixture as described above, and filling formed gelatin sheaths. Glidants and lubricants such as colloidal silica, talc, magnesium stearate, calcium stearate or solid polyethylene glycol can be added to the powder mixture before the filling operation. A disintegrating or solubilizing agent such as agar-agar, calcium carbonate or sodium carbonate can also be added to improve the availability of the medicament when the capsule is ingested.

In addition to the TAC, acceptable carriers and/or diluents may be employed and are familiar to those skilled in the art. Formulations in the form of pills, capsules, microspheres, granules or tablets may contain, in addition to one or more TACs, diluents, dispersing and surface-active agents, binders and lubricants. One skilled in the art may further formulate the TAC in an appropriate manner, and in accordance with accepted practices, such as those disclosed in Remington's Pharmaceutical Sciences, Gennaro, Ed., Mack Publishing Co., Easton, Pa., 1990. Moreover, when desired or necessary, suitable binders, lubricants, disintegrating agents and coloring agents can also be incorporated into the mixture. Suitable binders include starch, gelatinr, natural sugars such as glucose or beta-lactose, corn sweeteners, natural and synthetic gums such as acacia, tragacanth, or sodium alginate, carboxymethylcellulose, polyethylene glycol, waxes and the like. Lubricants used in these dosage forms include sodium oleate, sodium stearate, magnesium stearate, sodium, benzoate, sodium acetate, sodium chloride and the like. Disintegrators include, without limitation, starch, methyl cellulose, agar, bentonite, xanthan gum and the like. Tablets are formulated, for example, by preparing a powder mixture, granulating or slugging, adding a lubricant and disintegrant and pressing into tablets. A powder mixture is prepared by mixing the compound, suitably comminuted, with a diluent or base as described above, and optionally, with a binder such as carboxymethylcellulose, an aliginate, gelatin, or polyvinyl pyrrolidone, a solution retardant such as paraffin, a resorption accelerator such as a quate nary salt and/or an absorption agent such as bentonite, kaolin or dicalcium phosphate. The powder mixture can be granulated by wetting with a binder such as symp, starch paste, acadia mucilage or solutions of cellulosic or polymeric materials and forcing through a screen. As an alternative to granulating, the powder mixture can be run through the tablet machine and the result is imperfectly formed slugs broken into granules. The granules can be lubricated to prevent sticking to the tablet forming dies by means of the addition of stearic acid, a stearate salt, talc or mineral oil. The lubricated mixture is then compressed into tablets. The compounds of the present invention can also be combined with free flowing inert carrier and compressed into tablets directly without going through the granulating or slugging steps. A clear or opaque protective coating consisting of a sealing coat of shellac, a coating of sugar or polymeric material and a polish coating of wax can be provided. Dyestuffs can be added to these coatings to distinguish different unit dosages.

Oral fluids such as solution syrups and elixirs can be prepared in dosage unit form so that a given quantity contains a predetermined amount of the compound. Syrups can be prepared by dissolving the compound in a suitably flavored aqueous solution, while elixirs are prepared through the use of a non-toxic alcoholic vehicle. Suspensions can be formulated by dispersing the compound in a non-toxic vehicle. Solubilizers and emulsifiers such as ethoxylated isostearyl alcohols and polyoxy ethylene sorbitol ethers, preservatives, flavor additive such as peppermint oil or natural sweeteners or saccharin or other artificial sweeteners, and the like can also be added.

Where appropriate, dosage unit formulations for oral administration can be microencapsulated. The formulation can also be prepared to prolong or sustain the release as for example by coating or embedding particulate material in polymers, wax or the like.

The compound for use according to the present invention can also be administered in the form of liposome delivery, systems, such as small unilamellar vesicles, large unilamellar vesicles and multilamellar vesicles. Liposomes can be formed from a variety of phospholipids, such as cholesterol, stearylamine or phosphatidylcholines.

The compound may also be co-administered with soluble polymers as excipients or drug carriers. Such polymers can include polyvinylpyrrolidone, pyran copolymer, polyhydroxypropylmethacrylamidephenol, polyhydroxyethylaspartamidephenol, or polyethyleneoxidepolylysine substituted with palmitoyl residues. Furthermore, the compounds may be coupled to a class of biodegradable polymers useful in achieving controlled release of a drug, for example, polylactic acid, polepsilon caprolactone, polyhydroxy butyric acid, polyorthoesters, polyacetals, polydihydropyrans, polycyanoacrylates and cross-linked or amphipathic block copolymers of hydrogels.

Parenteral administration can be effected by utilizing liquid dosage unit forms such as sterile solutions and suspensions intended for subcutaneous, intramuscular or intravenous injection. These are prepared by suspending or dissolving a measured amount of the compound in a non-toxic liquid vehicle suitable for injection such as aqueous oleaginous medium and sterilizing the suspension or solution.

Alternatively, a measured amount of the compound is placed in a vial and the vial and its contents are sterilized and sealed. An accompanying vial or vehicle can be provided for mixing prior to administration. Non-toxic salts and salt solutions can be added to render the injection isotonic. Stabilizers, preservations and emulsifiers can also be added.

Rectal administration can be effected utilizing suppositories in which the compound is admixed with low-melting water-soluble or insoluble solids such as polyethylene glycol, cocoa butter, higher ester as for example flavored aqueous solution, while elixirs are prepared through myristyl palmitate or mixtures thereof.

Topical formulations of the present invention may be presented as, for instance, ointments, creams or lotions, eye ointments and eye or ear drops, impregnated dressings and aerosols, and may contain appropriate conventional additives such as preservatives, solvents to assist drug penetration and emollients in ointments and creams. The formulations may also contain compatible conventional carriers, such as cream or ointment bases and ethanol or oleyl alcohol for lotions. Such carriers may be present as from about 1% up to about 98% of the formulation. More usually they will form up to about 80% of the formulation.

For administration by inhalation the compound for use according to the present invention is conveniently delivered in the form of an aerosol spray presentation from pressurized packs or a nebulizer, with the use of a suitable propellant, e.g. dichlorodifluoromethane, trichlorofluoromethane, dichlorotetrafluoroethane, tetrafluoroethane, heptafluoropropane, carbon dioxide or other suitable gas. In the case of a pressurized aerosol the dosage unit may be determined by providing a valve to deliver a metered amount. Capsules and cartridges of e.g. gelatin for use in an inhaler or insufflator may be formulated containing a powder mix of a compound of the invention and a suitable powder base such as lactose or starch.

The drug for use in the composition of the present invention is beclomethasone dipropionate. Beclomethasone dipropionate has the following characteristics: rapid first-pass metabolism in the intestine and liver, low systemic bioavailability, high topical activity, and rapid excretion (see, e.g., Thiesen et al., Alimentary Pharmacology & Therapeutics 10:487-496, 1996).

The drug is beclomethasone dipropionate (BDP), on account of its very high topical anti-inflammatory activity. This drug can therefore be used effectively in very small doses, in the compositions of this invention, and will not enter the systemic circulation to any significant extent. BDP is a compound, which is available from a number of commercial sources, such as Schering-Plough Corporation (Kenilworth, N.J.) in bulk crystalline form, and has the following structure (i.e., beclomethasone 17,21-dipropionate):

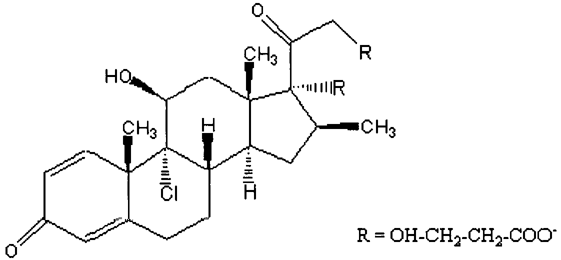

Patients receive a therapeutically acceptable amount of a TAC by oral administration. Suitable capsules or pills generally contain from 0.1 mg to 8 mg TAC, and typically about 1 mg TAC, plus optional fillers, such as lactose, and may be coated with a variety of materials, such as cellulose acetate phthalate.

In the context of inflammation caused by radiation , therapeutic administration of a TAC begins may begin with a larger dose of TAC steroid, which then may be tapered down to a maintenance dose of TAC after the inflammation has been controlled. In severe cases, more potent systemic steroids may be utilized to control the inflammation, then the patient may be quickly tapered onto a TAC steroid, or used in conjunction with formation of epithelial growth factors or cytokines.

An important aspect of this invention is that the TAC is orally administered such that it is topically administered to the intestinal or oral tissue. Thus, oral administration, as that term is used herein, is not intended to encompass systemic administration, such as by intravenous injection. Rather, the TAC has little (if any) systemic availability, but high topical activity on intestinal and/or liver tissue. The high topical activity is achieved by any of a number of means, known to those in the art, of limiting the distribution of the TAC to the intestinal mucosa. For example, the TAC may be formulated so as to coat the surface of the intestinal mucosa with a high local concentration of the TAC, or formulated so as to inhibit traversal of the drug across the intestinal mucosal into the systemic circulation. Such limited distribution results in fewer side effects, which is a significant advantage of this invention.

In treatment, the objectives are to suppress a wide variety of biological events that have already resulted in tissue destruction, for example, the generation of inflammatory cytokines, the recruitment of additional inflammatory cells to the site of injury, the destruction of the barrier function of the intestinal mucosa (the lining), the passage of bacteria and toxins through the damaged intestinal mucosa, the up-regulation of biologic responses to bacteria and endotoxin, and the widespread organ responses to these events.

By appropriate formulation of the TAC, it can be delivered to the entire mucosal surface of the entire intestine in high doses. Thus, the TAC can achieve high concentrations throughout the intestinal mucosa or oral mucosa, where this initiating inflammatory immune reaction is taking place.

### Examples

### Example 1 (reference). Supportive care with antibiotic therapy and treatment of the hematopoietic compartment after irradiation in dogs

The dog is a particularly suitable animal model for studying radiations damage to epithelial tissue. Death from the GI radiation syndrome was observed in dogs that were given myeloablative unfractionated total body irradiation (TBI) at a high dose rate (either 0.4 or 0.7 Gy/min). To evaluate the response to TBI given at the high dose rate of 0.4 Gy/min or 0.7 Gy/min, treatment groups were given single-fraction 6, 7, 8 or 10 Gy TBI and received previously cryopreserved autologous marrow infusion upon completion of the TBI and standard supportive care. At doses of TBI below 8 Gy, all dogs survived. After 8 Gy TBI, 5 of 9 dogs died and after 10 Gy TBI, 9 of 9 dogs died from GI radiation syndrome. All deaths occurred between days 4 to day 6 after 10 Gy TBI. All deaths were attributable due to GI radiation toxicity with severe gut hemorrhage and intestinal crypt damage, as well as denudation of gut epithelium and focal necrosis in the intestines. Despite antibiotic treatment, blood cultures obtained near the time of death were positive for gram negative organisms in 4 dogs, consistent with entry of pathogens through the GI tract. Among the 4 dogs that survived 8 Gy TBI, all had decreases in their blood cell counts but recovered hematopoiesis (since they were given autologous bone marrow), with recovery of neutrophil counts at 9-10 days after TBI and platelet counts at 16-20 days after TBI. There was no difference in outcome between the 0.4 and 0.7 Gy/min groups. When the TBI dose rate was reduced to 0.05 Gy/min or 0.02 Gy/min, dogs tolerated single-fraction TBI doses to 14 and 16 Gy without any GI toxicity. Fractionation of TBI also avoided the GI radiation syndrome and death.

For the purpose of identifying effective therapy for victims of a radiation terrorist attack or radiation therapy, it is important to consider the GI syndrome in the context of the hematopoietic syndrome following acute irradiation. The dog model of 10 Gy TBI given at a high dose rate followed with autologous bone marrow support permits the experimental system to examine the GI syndrome component of radiation injury. Autologous marrow infusion leads to more rapid recovery of neutrophil and platelet counts. By minimizing the impact of the hematopoietic syndrome with autologous marrow infusion, drugs that can directly treat the GI radiation syndrome can be studied. High-dose TBI administration to generate the GI radiation syndrome rather than focal irradiation of the GI tract is a more realistic scenario for modeling the systemic biologic response to a radiation terrorist attack.

Supportive care is critical for survival after radiation and that the cytokines granulocyte colony stimulating factor (G-CSF) and flt-3 ligand (FL) given in combination result in a significantly improved and rapid recovery of hematopoiesis after 7 Gy TBI and even after 8 Gy TBI. The importance of supportive care given after irradiation is evident when comparing the historical results of TBI survival studies from the 1980's and early 1990's. Historical results showed that after 4 Gy TBI (0.07 Gy/min, single fraction) and supportive care, only 1 of 28 dogs survived with recovery of hematopoiesis.

Supportive care consisted of the following: **(a)** intravenous antibiotics, ampicillin and amikacin and oral nonreabsorbable polymixin/neomycin administered without change in drug regimen until recovery of absolute neutrophil count (ANC)> 1000/µL; **(b)** transfusions with irradiated whole blood (50 mL blood per transfusion) for platelet counts below 10,000/µL; and **(c)** subcutaneous fluids 20 ml/kg given daily for the first 5 days after TBI.

After TBI exposure, all dogs were treated with an oral fluoroquinolone, enrofloxacin. Core body temperature was measured at least twice daily. If fever developed or when ANC falls below < 100/µL, dogs are empirically treated with the combination of intravenous third-generation cephalosporin (ceftazidime) and the aminoglycoside amikacin. Blood cultures are obtained when dogs develop fever and antibiotic treatment is adjusted based on blood culture results. If blood culture negative neutropenic fever persists for 48 hours, or if clinical condition objectively worsens, additional empiric antibiotic treatment with metronidazole and vancomycin is added. Although the new antibiotic regimen is more complicated compared with the historical treatment, it reflects improved broad spectrum coverage against gram negative, gram positive and anaerobic pathogens. Furthermore, blood transfusion support is more intensive now with larger volume of whole blood transfusion, based on body weight with 10-15 mL blood/kg/transfusion. The use of intravenous fluid support (10-30 mL/kg/day Lactated Ringers solution) is extended for at least 14 additional days until there is full recovery of oral food intake.

New supportive care measures have shown a significant improvement in survival after TBI. Following 4 Gy TBI (0.07 Gy/min, Varian Clinac 4/80 linear accelerator source), 4 of 4 dogs survived and recovered hematopoiesis with a median time to ANC and platelet recovery of 27 and 41 days, respectively. ANC recovery was defined as the first day of sustained neutrophil counts > 500/ µL and platelet recovery was defined as the first day of transfusion-independent sustained recovery of > 40,000/µL. After 5 Gy TBI, 3 of 6 dogs survived with supportive care only. Deaths occurred at days 14, 15 and 21, respectively, after TBI and were due to neutropenic sepsis or pneumonia and not from the GI radiation syndrome. Among surviving dogs, the median time to ANC and platelet recovery was 29 and 44 days, respectively. After 6 Gy TBI, 5 of 6 dogs survived given supportive care only. One death occurred on day 22 due to neutropenic sepsis. The median time to ANC and platelet recovery was 34 and 74 days, respectively. After 7 Gy TBI and supportive care only, 5 of 6 dogs survived, with one death on day 22 due to neutropenic sepsis. The median time to ANC recovery was 43 days, and platelet recovery was achieved at 53 and 62 days in two dogs, respectively. As of April 18, 2007, the remaining three dogs in this cohort remain transfusion-dependent at 66 to 95 days after TBI. These preliminary results show that intensive supportive care with multiple empiric broad spectrum antibiotics during the period of neutropenia, sustained transfusion support, and aggressive early intravenous fluid support is critical for the survival after TBI. Because of the improved survival of dogs after TBI given optimal supportive care in the current AI-066498 studies, we have not yet reached the 99% lethal dose of TBI threshold.

Although supportive care without cytokine support results in improved survival, cytokine treatment given after TBI aimed at recovery of the hematopoietic system also improves outcome and decreased the need for prolonged, intensive (and expensive) supportive care. Historical results in the dog model by our research group included studies which established that both recombinant canine (rc) and recombinant human (rh) G-CSF given after an otherwise lethal dose of 4 Gy TBI prevented death from radiation induced cytopenia. In the 1980's and early 1990's, after 4 Gy TBI and supportive care alone, only 1 of 28 dogs survived with recovery of hematopoiesis. In contrast, when G-CSF was given daily for 21 days after 4 Gy TBI, 8 of 10 dogs survived. Following 5 Gy TBI, G-CSF treatment resulted in survival of 3 of 10 dogs. These studies helped to establish the current clinical guidelines for the treatment of victims of radiation accidents.

G-CSF and FL given after TBI shows an improved and more rapid recovery of ANC and platelet counts compared with supportive care alone. Following 5 Gy TBI and daily treatment with G-CSF (10 µg/kg/day) starting 2 hours after completion of irradiation, 6 of 6 dogs survived. The median time to ANC and platelet recovery was 20 and 44 days, respectively, suggesting that G-CSF promoted more rapid recovery of neutrophils compared with supportive care alone. After 6 Gy TBI and G-CSF treatment, 5 of 6 dogs survived and the median time to ANC and platelet recovery was 26 and 61 days, respectively. One dog was euthanized on day 77 due to failure to recover ANC > 500/µL. After 6 Gy TBI and treatment with the combination of G-CSF (10 µg/kg/day) and FL (100 µg/kg/day), starting 2 hours after TBI and continuing until recovery of ANC> 1000/µL, 5 of 5 dogs survived. The median time to ANC and platelet recovery was 20 and 47 days, respectively. Treatment with G-CSF and FL showed significantly improved neutrophil and platelet recovery compared with supportive care alone. Following 7 Gy TBI and G-CSF plus FL, 6 of 6 dogs survived and the median time to ANC and platelet recovery was 20 and 56 days, respectively. Treatment with G-CSF and FL showed a more substantial improvement in neutrophil and platelet recovery compared to supportive care alone after the higher dose of 7 Gy TBI. After discontinuation of G-CSF and FL, peripheral blood counts remained stable and continued to return to normal levels. Dogs were followed until 6 months after TBI with evaluation of immune function recovery. To date, at 6 months after TBI, dogs have recovered normal immune function based on T and B cell responses to neoantigen in vivo and in vitro.

### Example 2 (reference). Treatment of radiation injury with KGF

The canine KGF gene has been cloned and sequenced. RhKGF has 97.4% amino acid sequence homology to canine KGF. The murine KGF protein has 94% homology to rhKGF. In addition, the KGF receptor (FGFR2IIIb) has 98% homology between all three species. RhKGF was used for all of the in vivo murine studies described above. rhKGF has equivalent biologic activity in dogs. RhKGF has a gut epithelial cytoprotective activity in the dog. KGF treatment decreases gut, thymic and bone marrow stromal epithelial damage from radiation, thereby improving survival after TBI induced GI toxicity and pancytopenia with more rapid intestinal epithelium recovery and immune reconstitution.

Three non-irradiated dogs were given rhKGF 100 µg/kg/day for 7, 14 and 21 days, respectively. Twenty-four hours after the last dose of KGF, dogs were euthanized and underwent complete necropsy. Among the dogs that received KGF for 14 and 21 days, there was a dramatic increase in the size and cellularity of Peyer's patches throughout the ileum and a dramatic increase in size of villi in the jejunum.

To evaluate the capacity of KGF to protect against high dose total body irradiation (TBI), KGF was given at a dose of 100 µg/kg/day intravenously starting 2 hours after TBI the dose of TBI that resulted in > 80% lethality and was given daily until full clinical recovery of GI tract function. Dogs were monitored for survival up to 30 days after initiation of TBI (primary endpoint of the experiment). This dose of KGF provided ≥ 70% survival after the dose of TBI. Dogs were evaluated in cohorts of three dogs to identify drug treatment that significantly improves survival after TBI and proceeded to larger groups of up to 12 dogs per treatment group to further evaluate statistical significance compared to the concurrent control group evaluating survival at day 30. Secondary endpoints included time to histologic and physiologic evidence of GI tract recovery. A subset of dogs underwent serial endoscopies to evaluate the effect of drug treatment both histologically and by molecular evaluation of wnt/b catenin and Notch signaling. Dogs were also followed with daily CBCs until recovery and assessed for immune function recovery. Supportive care measures and autologous marrow infusion were given simultaneously with the evaluation of the combination of KGF and BDP.

### Example 3. Treatment of radiation injury with BDP

As in example 2, BDP was given at a dose of 4 mg/day from 2 hours after TBI until GI recovery and the same experimental endpoints were monitored. BDP provided greater than 70% survival.

### Example 4. Concomitant treatment of radiation injury with KGF and BDP

Supportive care measures and autologous marrow infusion were given simultaneously with the evaluation of the combination of KGF and BDP. Oral BDP (orBec) was given at a dose 4 mg/day concurrently with KGF dosed 100 µg/kg/day intravenously, starting 2 hours after TBI the dose of TBI that resulted in > 80% lethality from 2 hours after TBI until GI recovery. Dogs were monitored for adrenal suppression.

### Example 5 (reference). Treatment of radiation injury with Lithium carbonate

Lithium carbonate was dosed at 300 mg/day to achieve steady state serum therapeutic level of 0.7-1.4 mEq/L [34] and monitored with weekly levels. TBI was initiated when steady state of lithium was reached, and dogs were monitored for survival and other endpoints as described in example 1.

### Example 6. Concomitant treatment of radiation injury with lithium carbonate and BDP.

Lithium carbonate was dosed at 300 mg/day to achieve steady state serum therapeutic level of 0.7-1.4 mEq/L [34] and monitored with weekly levels. Concurrent BDP was given at a dose of 4 mg/day. TBI was initiated when steady state of lithium was reached, and dogs were monitored for survival and other endpoints as described in example 1.

## Claims

1. Pharmaceutical composition for use in preventing, ameliorating and/or treating damage to the lining of the alimentary tract of a patient by administering an effective amount of a topically active corticosteroid, wherein
the damage is a result of the patient's exposure to total body irradiation,
the topical active corticosteroid is beclomethasone dipropionate,
the effective amount of beclomethasone dipropionate is 4 mg/day, and
the administration of beclomethasone dipropionate is carried out from 2 hours after the total body irradiation.

2. The pharmaceutical composition for use of claim 1, where the topically active corticosteroid is administered in combination with a second compound that is intended to treat another cellular aspect of tissue damage, including growth factors, regulator molecules, anti-inflammatory molecules including keratinocyte growth factor (KGF), R-spondin-1 and its analogues and homologues, hormones including somatostatin, octreotide, gastrin, Ghrelin, inhibitors of cyclo-oxygenase-2, antioxidants, vitamin E, sucralfate, analogues of lysophophatidic acid and agonists or antagonists of LPA-2 receptor, amifostine, and other radioprotectant or radio-mitigating drugs.

## Patentansprüche

1. Pharmazeutische Zusammensetzung zur Verwendung bei der Vorbeugung, Linderung und/oder Behandlung von Schäden an der Auskleidung des Verdauungstraktes eines Patienten durch Verabreichung einer wirksamen Menge eines topisch aktiven Kortikosteroids, wobei
der Schaden eine Folge der Exposition des Patienten gegenüber einer Ganzkörperbestrahlung ist,
das topisch aktive Kortikosteroid Beclometasondipropionat ist,
die wirksame Menge an Beclometasondipropionat 4 mg/Tag beträgt und
die Verabreichung von Beclometasondipropionat ab 2 Stunden nach der Ganzkörperbestrahlung erfolgt.

2. Pharmazeutische Zusammensetzung zur Verwendung nach Anspruch 1, wobei das topisch aktive Kortikosteroid in Kombination mit einer zweiten Verbindung verabreicht wird, die dazu bestimmt ist, einen anderen zellulären Aspekt der Gewebeschädigung zu behandeln, einschließlich Wachstumsfaktoren, Regulatormolekülen, entzündungshemmenden Molekülen einschließlich Keratinozytenwachstumsfaktor (KGF), R-Spondin-1 und seine Analoga und Homologe, Hormone einschließlich Somatostatin, Octreotid, Gastrin, Ghrelin, Inhibitoren der Cyclooxygenase-2, Antioxidantien, Vitamin E, Sucralfat, Analoga der Lysophosphatidsäure und Agonisten oder Antagonisten des LPA-2-Rezeptors, Amifostin und andere Strahlenschutzmittel oder verstrahlungsmindernde Mittel.

## Revendications

1. Composition pharmaceutique destinée à être utilisée pour prévenir, améliorer et/ou traiter une lésion de la muqueuse du tube digestif d'un patient en administrant une quantité efficace d'un corticostéroïde topiquement actif,
dans lequel,
- la lésion résulte de l'exposition à une irradiation totale du corps,
- le corticostéroïde topiquement actif est le dipropionate de béclométhasone ;
- la quantité efficace de dipropionate de béclométhasone est de 4 mg/jour et
- l'administration de dipropionate de béclométhasone n'est effectuée à partir de 2 heures depuis l'irradiation totale du corps.

2. La composition pharmaceutique pour une utilisation selon la revendication 1, dans laquelle le corticostéroïde actif par voie topique est administré en association avec un second composé destiné à traiter un autre aspect cellulaire des lésions tissulaires incluant des facteurs de croissance, des molécules régulatrices, des molécules anti-inflammatoires incluant le facteur de croissance des kératinocytes (KGF), de la R-spondin-1 et ses analogues et homologues, des hormones incluant la somatostatine, l'octréotide, la gastrine, la ghréline, les inhibiteurs de la cyclo-oxégénase-2, les antioxydants, la vitamine E, le sucralfate, les analogues de l'acide lysophosphatidique et les agonistes ou antagonistes de récepteurs de la LPA-2, l'amifsotine et autres médicaments radioprotecteurs ou radio-atténuants.
